# EUROPEAN PATENT SPECIFICATION

(11) **EP 3 008 456 B1**
(45) Date of publication and mention of the grant of the patent: **14.08.2024**
(21) Application number: 14788649.3
(22) Date of filing: 28.04.2014
(51) Int. Cl.: G01N 23/083, A61B 6/03, A61B 6/04, A61B 6/10, A61B 6/42, A61B 6/00, H05K 1/02, H05K 1/18

(54) **ACTIVE SHIELD FOR X-RAY IMAGING**
AKTIVE ABSCHIRMUNG ZUR RÖNTGENBILDGEBUNG
BOUCLIER ACTIF POUR IMAGERIE PAR RAYONS X

(30) Priority: 26.04.2013 US 201361816244 P; 07.02.2014 US 201414175344
(43) Date of publication of application: 20.04.2016
(73) Proprietor: Texas Instruments Incorporated, Dallas, TX 75265-5474 (US)
(72) Inventor: KODURI, Sreeenivasan, K., Allen, TX 75013 (US)
(74) Representative: Zeller, Andreas
(86) International application number: PCT/US2014/035694
(87) International publication number: WO 2014/176594

(56) References cited:
- WO-A1-2013/130400
- SU-A1- 1 279 383
- US-A1- 2002 070 343
- US-A1- 2007 205 367
- US-A1- 2012 097 857
- US-A1- 2012 133 001
- US-B1- 6 304 626

## Description

### SUMMARY

A disclosed X-ray apparatus includes a radiation shielding substrate having a first side and an opposite second side; a wraparound electrical circuit wrapped around the substrate; an X-ray sensor assembly adapted to generate sensor signals indicative of X-rays impinged thereon, the sensor assembly being connected to the wraparound electrical circuit and positioned adjacent to the first side of the substrate; and a sensor signal processing assembly connected to the wraparound electrical circuit and positioned adjacent to the second side of the substrate The wraparound electrical circuit comprises a plurality of circuit traces, wherein each of the plurality of circuit traces has a length no longer than about 100 mm, and wherein the distance between the first and second surfaces of the substrate is between about 0.5 mm and 2 mm.

An active shield for an X-ray computed tomography machine ("CT machine") includes a radiation shielding substrate; and a wraparound electrical circuit wrapped around the substrate. The radiation shielding substrate has a first side and a second side, and the distance between the first and second surfaces of the radiation shielding substrate is between about 0.5 mm and 2 mm, and wherein the wraparound electrical circuit is adapted to route electrical signals along circuit traces having a length no longer than 100 mm from a sensor assembly positioned adjacent the first side of the substrate to signal processing electronics positioned adjacent the second side of the substrate.

A method of generating digital signals representative of an image of a subject includes transmitting X-rays through the subject from an X-ray source on one side of the subject to a sensor assembly supported on one side of an active shield having opposite first and second sides, the active shield being located on a side of the subject opposite to the side where the X-ray source is located; and routing analog signals generated by the sensor assembly along circuit traces having a length no longer than 100 mm to signal processing electronics supported on the side of the active shield opposite the side supporting the sensor assembly. The distance between the first and second surfaces of the active shield is between about 0.5 mm and 2 mm.

### BRIEF DESCRIPTION OF THE DRAWINGS

FIG. 1 (Prior Art) is an overall perspective view of an X-ray computed tomography (CT) machine.
FIG. 2 (Prior Art) is a schematic end elevation view of a conventional gantry of a CT machine.
FIG. 3 (Prior Art) is a schematic side elevation view of a conventional X-ray sensor assembly of a CT machine.
FIG. 4 (Prior Art) is a schematic isometric view of a scintillator of a conventional X-ray sensor assembly.
FIG. 5 (Prior Art) is an isometric view showing the relationship between a conventional X-ray sensor assembly and signal processing electronics.
FIG. 6 is a schematic side elevation view of an active shield having an X-ray sensor assembly mounted on the first side thereof and signal processing electronics mounted on a second side thereof.
FIG. 7 is an isometric top view of an active shield.
FIG. 8 is an upside down cross-sectional view of active shield.
FIG. 9 is an isometric bottom view of an active shield and electronics mounted thereon.
FIG. 10 is a top plan view of one layer of a wraparound electrical circuit.
FIG. 11 is a partially cut away isometric view of another embodiment of an active shield.

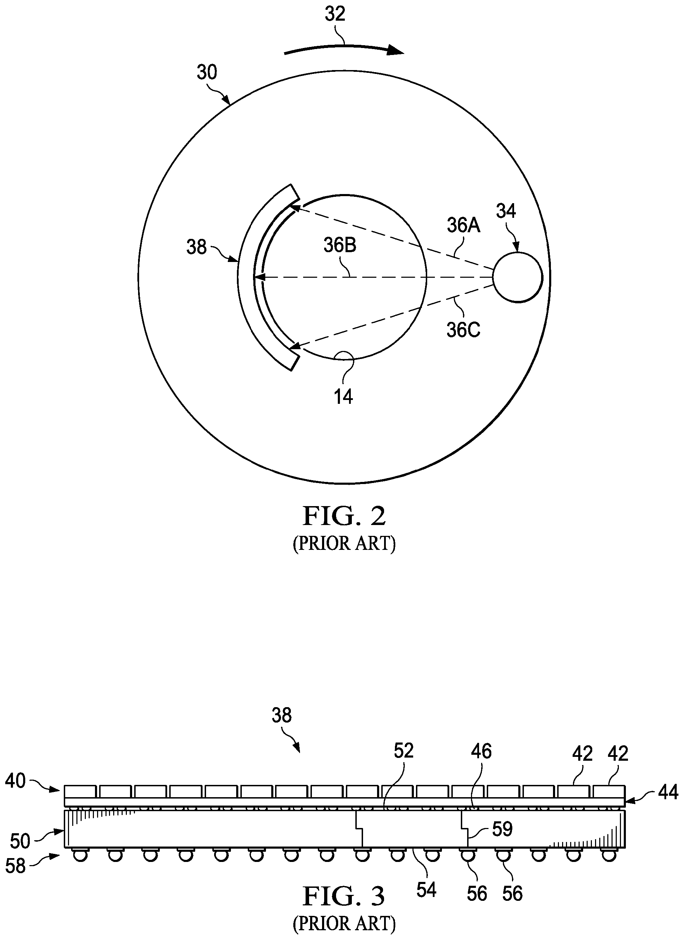

FIG. 2 is schematic end elevation view of the gantry of a CT machine.

### DETAILED DESCRIPTION OF EXAMPLE EMBODIMENTS

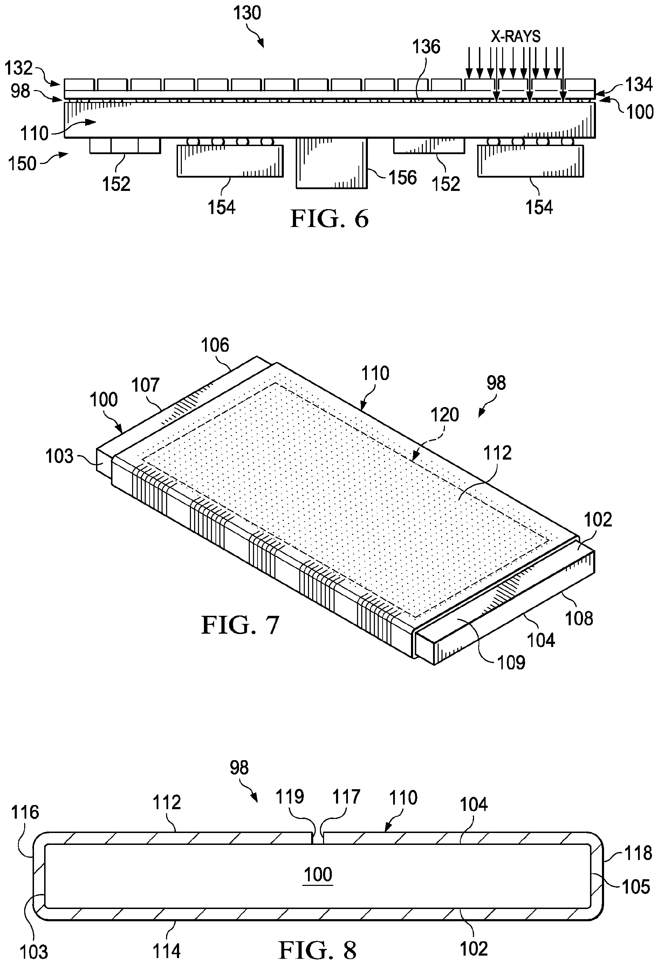

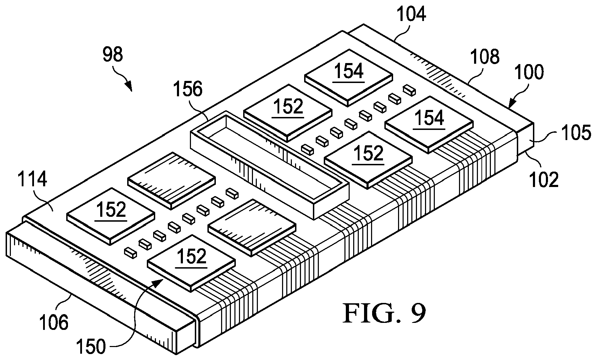

In general, an X-ray computed tomography machine 210 ("CT machine") is described herein. As best shown in FIGS. 6-9 and 12, the CT machine 210 has a radiation shielding substrate 100 with a first side 102 and an opposite second side 104. It includes a wraparound electrical circuit 110 that is wrapped around the radiation shielding substrate 100. The CT machine also has an X-ray sensor assembly 130 that is adapted to generate sensor signals indicative of X-rays impinged on it. The sensor assembly 130 is connected to the wraparound electrical circuit 110 and is positioned adjacent to the first side 102 of the substrate. The CT machine also has a sensor signal processing assembly 150 which is connected to the wraparound electrical circuit 110. The signal processing assembly 150 is positioned adjacent to the second side 104 of the substrate 100. The signal processing assembly 150 is shielded from X-rays by the radiation shielding substrate 100.

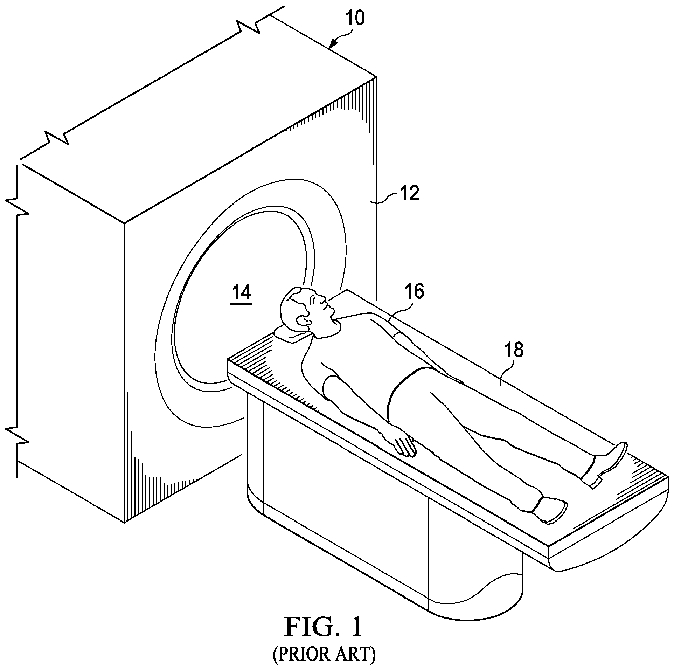

FIG. 1 illustrates a prior art X-ray computed tomography machine 10 (CT machine 10). The CT machine 10 has a CT housing 12 with a center opening 14 therein. During CT operation, a patient 16 lies on a horizontally disposed table 18. The table 18 is slowly moved through the central opening 14 as X-rays from the machine pass through the patient's body.

FIG. 2 is a schematic view of a gantry 30 of the prior art CT machine 10. The gantry 30 is a massive doughnut-shaped device that is positioned behind a front cover of the CT machine 10. The gantry 30 rotates in direction 32 as the patient is moved through the opening 14. The gantry 30 includes an X-ray tube 34 positioned on one side of the central hole 14. An X-ray sensor assembly 38 is positioned on the opposite side of the center opening 14. X-rays 36A, 36B, 36C from the X-ray tube 34 are sensed by the sensor assembly 38. Analog signals from the sensor assembly 38 are processed and used to create three-dimensional images of the interior of the patient's body.

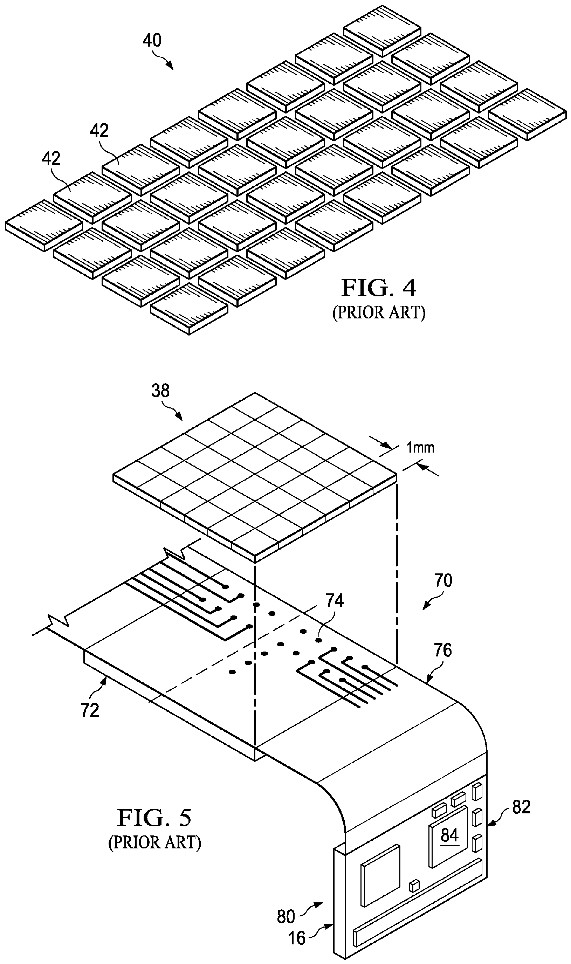

FIG. 3 is a schematic side elevation view of a portion of the prior art X-ray sensor assembly 38. Sensor assembly 38 includes a scintillator array 40 mounted on a photo detector array 44 that is, in turn, mounted on a ceramic substrate 50. FIG. 4 is an enlarged isometric view of a top portion the scintillator array 40, which comprises a grid of scintillator pixels 42. As shown by FIG. 3, the scintillator array 40 is supported on a photo detector array 44 having a plurality of pixels (not shown individually) corresponding to the pixels 42 of the scintillator array 40. (The term "pixel" as used herein may refer to an element of an image sensor as well as an element of an image.) The scintillator and photo detector pixels need to be held with high levels of geometric tolerances against mechanical, thermal, gravitational, and aging effects. Typically a thick ceramic substrate 50 is used to provide the necessary support. The ceramic substrate 50 has flat upper and lower surfaces 52, 54. A plurality of conductor pads (not shown) are provided on the top surface 52 of substrate 50 in alignment with conductors 46 on the photo detector assembly 44. Electrical contacts such as balls 56 of a ball grid array 58 are provided on the bottom surface 54 of the ceramic substrate 50. Conductors 59 extending through vias in the substrate 50 connect conductor pads (not shown) on the top surface 52 to corresponding ball conductors 56 on the bottom surface 54.

As illustrated by FIG. 5, the X-ray sensor assembly 38 is connected to signal processing electronics 80 which process analog signals from the photo detector array 44. The analog signals from the photo detector array can be corrupted if transmitted over long distances. To avoid such signal corruption the analog signals would ideally be filtered and digitized by electronics in close proximity to the photo detector 44. The digital signals thus produced could then be safely transmitted, stored, and further processed with no loss of integrity.

X-rays that strike the scintillator array 40 are absorbed by it. However some amount of radiation may escape from the sensor assembly 38 due to gap's between scintillator pixels 42, or because some X-rays pass entirely through the sensor assembly 38. Such stray X-rays can significantly alter the characteristics of signal processing electronics placed in the proximity of the sensor assembly 38.

Various techniques have evolved to prevent damage to the signal processing electronics. US 2012/097857 A1 discloses a radiation detection unit for X-ray computed tomography apparatus, which has an integrated circuit that is arranged on the sides of a radiation shielding structure by folding the substrate along the outer edges of the shielding structure. Other techniques generally involve use of additional printed circuit boards (PCB's) to route the signals to signal processing electronics located a substantial distance away from the sensor assembly. An example of such a prior art assembly is shown in FIG. 5. An analog signal transfer assembly 70 transfers analog signals from the X-ray sensor assembly 38 to a signal processing assembly 80. The analog signal transfer assembly 70 includes a first conventional PCB 72. The ball grid array 58 on the ceramic substrate 50 is placed in electrical contact with a corresponding conductor array 74 on the conventional PCB 72. A flexible PCB 76 connects the first conventional PCB 72 to a second conventional PCB 82 having analog signal processing electronics 84 mounted on it. A portion of the flexible PCB 76 and the second conventional PCB 82 extend generally perpendicular to the first conventional PCB 72 and parallel to the direction of X-rays from the X-ray tube 34. Although this structure places the signal processing electronics 82 at a distance from the source of the X-rays it has a number of drawbacks. Such structure undesirably increases the distance that the analog signals must travel to reach the signal processing electronics. Such structure also adds undesirable stack height (radial height) and mass to the entire X-ray sensor/signal processing assembly. Because of the rapid rotation of the gantry, significant centrifugal forces are generated that cause such structures to be mechanically unstable and electrically unreliable.

FIG. 6 illustrates an X-ray sensing and signal processing assembly that may be used to overcome the above described problem in CT machines. An X-ray sensor assembly 130 is mounted on one side of an active shield assembly 98. The X-ray sensor assembly 130 may comprise a scintillator array 132 and a photo detector array 134, which may be the same as the prior art scintillator array 40 and photo detector array 44 described above. Signal processing electronics 150 are positioned on the other side of the active shield assembly 98. The signal processing electronics 150 may comprise, for example, passive circuit devices 152 and integrated circuit packages 154. An electrical connector assembly 156 routes processed digital signals from the electronics 150 to other system electronics such as storage devices and displays (not shown).

Because the signal processing electronics 150 are positioned on the side of the active shield assembly 98 that is opposite the side facing the X-ray source, the electronics 150 are shielded from the X-rays. Also, because the electronics 150 are located close to the X-ray sensor assembly 130 the analog signals from the sensor assembly 130 travel only an advantageously short distance to the electronics 150. The analog signals are transmitted through a wraparound electric circuit 110, which is described below.

FIGS. 7-9 illustrate various features of the active shield assembly 98 shown in FIG. 6. The active shield assembly 98 comprises a radiation shielding substrate 100. The substrate 100 is constructed from a radiation blocking material such as tungsten or lead and has a first side 102 and opposite second side 104. The two sides 102 and 104 may comprise flat, generally parallel surfaces. The two sides 102, 104 are connected by smaller lateral sides 103, 105. The substrate 100 has a first longitudinal end 106 and a second longitudinal end 108. A typical thickness range for the substrate 100 is about 0.5 mm to about 2.0 mm. Thus the X-ray sensor assembly 130 may be positioned less than about 3mm from the signal processing electronics 150. An advantage of using a tungsten substrate 100, in addition to its excellent radiation shielding characteristics, is that it has a very low coefficient of thermal expansion. This helps in creating a very rigid, flat surface that does not change dimensions much with temperature. Thus a stable surface is provided for supporting the X-ray sensor assembly 130.

The active shield assembly 98 also comprises a wraparound electrical circuit 110, an embodiment of which is shown in Figs 7-10. The wraparound circuit 110 comprises a first larger portion 112 that engages and substantially covers the first side 102 of the substrate 100 and a second larger portion 114 that engages and substantially covers the second side 104 of the substrate 100. The wraparound circuit 110 also comprises third and fourth smaller portions 116,118 that engage and substantially cover the small lateral side portions 103, 105 of the substrate 100. Wrap around electrical circuit 110 has terminal end portions 117, 119, which are positioned adjacent one another when the circuit 110 is wrapped around the substrate 100, as that shown in FIG. 8.

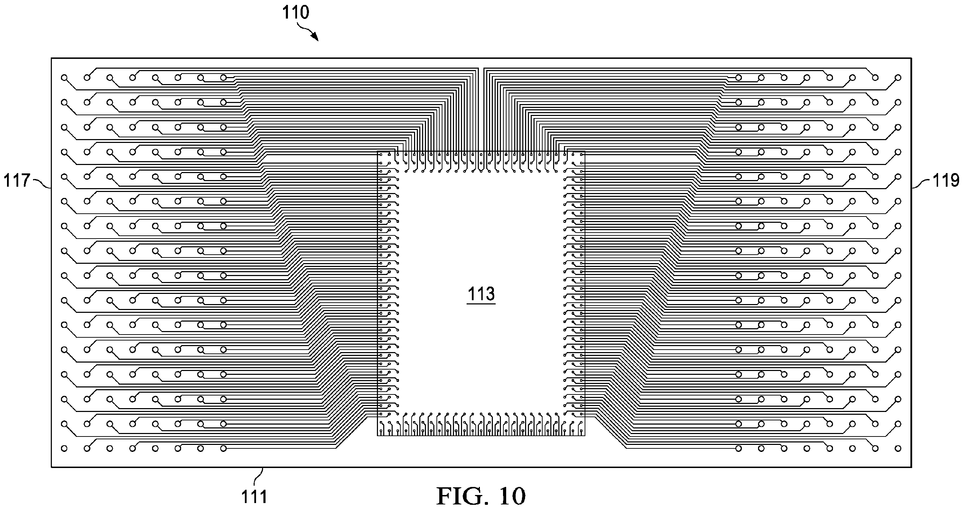

One layer of this flexible printed circuit board ("flex PCB") type wraparound electrical circuit 110 is illustrated in FIG. 10. It includes a sheet of flexible nonconductive material 111, such as Kapton, polymer, etc., having a plurality of copper traces 113 formed on it. The traces 113 route analog signals from the photo detector assembly 134, which is located on one side 102 of the radiation blocking substrate 100, to the signal processing electronics 150 on the other side 104. In one embodiment each of the traces has a length no longer than about 100 mm. The void 115 in the center of the sheet 111 is the region where the signal processing electronics 150 would be located. The wrap around electrical circuit 110 may have one layer or more than one layer of the type illustrated in FIG. 10. In a multiple layer wraparound electrical circuit structure the layers would be interconnected by vias. One of these layers can be used as an electrical ground to isolate the high-speed signals from each other and from the radiation shielding substrate 100. It is also possible to electrically connect the ground of the wraparound electrical circuit 110 to the substrate 100 to eliminate any induced eddy currents or electrostatic build up.

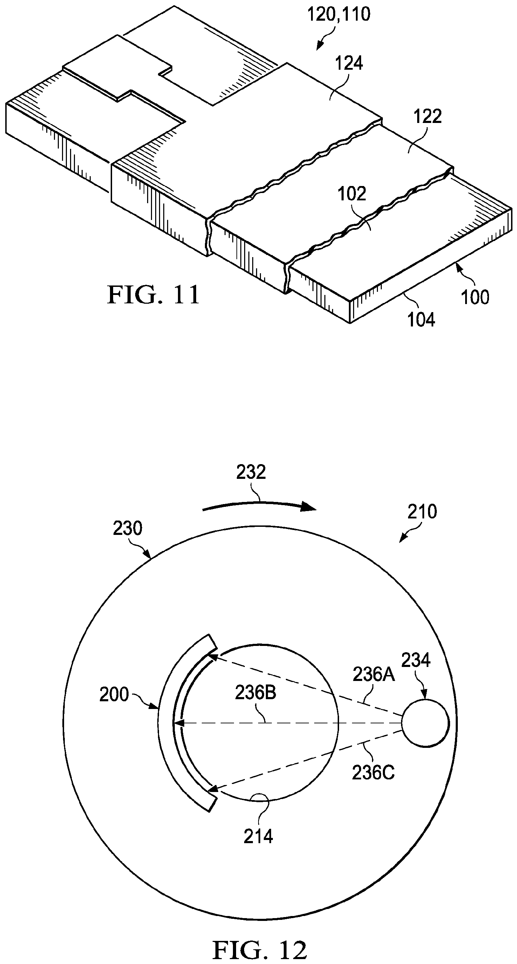

In another embodiment, as shown in FIG. 11, the wraparound electrical circuit 110 may be a metal clad structure 120. It includes a dielectric layer 122 that is adhered to the entire surface of the radiation shielding substrate 100. A patterned circuit layer 124 is provided on top of the dielectric layer 122, as by photolithography or other means.

As shown in FIG. 12, the active shield 98, X-ray sensor assembly 130 and sensor signal processing electronics 150 (referred to collectively as assembly 200) may be used in a CT machine 210. The CT machine 210 may have the same basic construction as the CT machine 10 described above with reference to FIGS. 1 and 2, except that assembly 200 replaces the X-ray sensor assembly 38 and the signal processing electronics associated therewith.

Multiple active shields 98 may be tiled together, to create a continuous larger active shield structure that supports a large X-ray sensor assembly 130 on one side and sensor signal processing electronics 150 on the other side. End portions of multiple substrates 100 could have features allowing them to be locked together, for example, dovetail joints. The wraparound electric circuit 110 maybe constructed to expose end portions 107, 109 of the substrate 100 to facilitate such locking connection.

It will be appreciated from the above disclosure that a method of generating digital signals representative of an image of a subject may include transmitting X-rays 236A, 236B, 236C through the subject from an X-ray source 234 on one side of the subject to a sensor assembly 130 supported on one side of an active shield 98 having opposite first and second sides, the active shield 98 being located on a side of the subject opposite to the side where the X-ray source 234 is located. The method may further include routing analog signals generated by the sensor assembly 130 to signal processing electronics 150 supported on the side of the active shield 98 opposite the side supporting the sensor assembly 130. The routing may comprise routing the analog signals through a wraparound circuit portion 110 of the active shield 98 that is wrapped around an X-ray blocking substrate portion 100 of the active shield 98.

Although the specific embodiment of a CT machine that is described herein is a medical CT machine, the CT machine features described herein are also applicable to other types of CT machines such as industrial CT machines used for imaging solder joints on printed circuit boards.

Those skilled in the art will appreciate that modifications may be made to the described embodiments, and also that many other embodiments exist, within the scope of the claimed invention.

## Claims

1. An X-ray apparatus, comprising:
a radiation shielding substrate (100) having a first side (102) and an opposite second side (104);
a wraparound electrical circuit (110) wrapped around the substrate;
an X-ray sensor assembly (130) adapted to generate sensor signals indicative of X-rays impinged thereon, the sensor assembly being connected to the wraparound electrical circuit and positioned adjacent to the first side (102) of the substrate; and
a sensor signal processing assembly (150) connected to the wraparound electrical circuit and positioned adjacent to the second side (104) of the substrate, wherein the wraparound electrical circuit comprises a plurality of circuit traces (113), wherein each of the plurality of circuit traces has a length no longer than about 100 mm, and wherein the distance between the first and second surfaces of the substrate is between about 0.5 mm and 2 mm.

2. The apparatus of claim 1 wherein the wraparound electrical circuit comprises a flexible printed circuit board.

3. The apparatus of claim 1 wherein the wraparound electrical circuit comprises a dielectric layer (122) formed on the radiation shielding substrate and a circuit layer (124) formed on the dielectric layer.

4. The apparatus of claim 1 wherein the wraparound electrical circuit substantially covers the first side and the second side of the substrate.

5. The apparatus of claim 1 wherein the sensor assembly comprises:
a scintillator array (132) that converts X-rays to light; and
a light detector array (134) that receives light from the scintillator array and converts it to an analog electrical signal.

6. The apparatus of claim 4 wherein the wrap around electrical circuit transmits the analog electrical signals from the light sensor array to the sensor signal processing electronics and wherein the sensor signal processing electronics coverts the analog electrical signals into digital signals.

7. The apparatus of claim 1 wherein the radiation shielding substrate is made from at least one of a metal and a metal alloy.

8. The apparatus of claim 1 wherein the radiation shielding substrate is made from tungsten.

9. The apparatus of claim 1 wherein the sensor assembly is positioned less than about 3 mm from the sensor signal processing electronics.

10. The apparatus of claim 1 wherein the flexible circuit board comprises a plurality of circuit layers.

11. The apparatus of claim 10 wherein one of the plurality of circuit layers comprises a ground layer.

12. The apparatus of claim 11 wherein the ground layer is connected to the radiation shielding substrate.

13. An active shield for an X-ray computed tomography machine ("CT machine") comprising:
a radiation shielding substrate (100); and
a wraparound electrical circuit (110) wrapped around the substrate, wherein the radiation shielding substrate has a first side and a second side , and the distance between the first and second surfaces of the radiation shielding substrate is between about 0.5 mm and 2 mm, and wherein the wraparound electrical circuit is adapted to route electrical signals along circuit traces having a length no longer than 100mm from a sensor assembly (200) positioned adjacent the first side of the substrate to signal processing electronics positioned adjacent the second side of the substrate.

14. The active shield of claim 13 wherein the substrate is adapted to mechanically support the sensor assembly and the signal processing electronics.

15. The active shield of claim 14 wherein the radiation shielding substrate is adapted to shield the signal processing electronics positioned adjacent the second side of the substrate from an X-ray source positioned on the first side of the substrate.

16. A method of generating digital signals representative of an image of a subject comprising:
transmitting X-rays (236A, 236B, 236C) through the subject from an X-ray source (234) on one side of the subject to a sensor assembly (130) supported on one side of an active shield (98) having opposite first and second sides, the active shield being located on a side of the subject opposite to the side where the X-ray source is located; and
routing analog signals generated by the sensor assembly along circuit traces of a wraparound circuit portion (110), the circuit traces having a length no longer than 100mm to signal processing electronics (150) supported on and positioned adjacent the side of the active shield opposite the side supporting the sensor assembly, and wherein the distance between the first and second surfaces of the active shield is between about 0.5 mm and 2 mm.

17. The method of claim 16 wherein the wraparound circuit portion of the active shield is wrapped around an X-ray blocking substrate portion of the active shield.

## Patentansprüche

1. Röntgengerät, aufweisend:
ein strahlungsabschirmendes Substrat (100) mit einer ersten Seite (102) und einer gegenüberliegenden zweiten Seite (104);
einen umlaufenden elektrischen Schaltkreis (110), der um das Substrat gewickelt ist;
eine Röntgensensorbaugruppe (130), die dazu ausgelegt ist, Sensorsignale zu erzeugen, die darauf auftreffende Röntgenstrahlen anzeigen, wobei die Sensorbaugruppe mit dem umlaufenden elektrischen Schaltkreis verbunden ist und neben der ersten Seite (102) des Substrats positioniert ist; und
eine Sensorsignalverarbeitungsbaugruppe (150), die mit dem umlaufenden elektrischen Schaltkreis verbunden ist und neben der zweiten Seite (104) des Substrats positioniert ist;
wobei der umlaufende elektrische Schaltkreis mehrere Leiterbahnen (113) aufweist, wobei jede der mehreren Leiterbahnen eine Länge von nicht mehr als etwa 100 mm aufweist, und
wobei der Abstand zwischen der ersten und der zweiten Fläche des Substrats zwischen etwa 0,5 mm und 2 mm beträgt.

2. Gerät nach Anspruch 1, wobei der umlaufende elektrische Schaltkreis eine flexible Leiterplatte aufweist.

3. Gerät nach Anspruch 1, wobei der umlaufende elektrische Schaltkreis eine dielektrische Schicht (122), die auf dem strahlungsabschirmenden Substrat gebildet ist, und eine Schaltkreisschicht (124), die auf der dielektrischen Schicht gebildet ist, aufweist.

4. Gerät nach Anspruch 1, wobei der umlaufende elektrische Schaltkreis im Wesentlichen die erste Seite und die zweite Seite des Substrats bedeckt.

5. Gerät nach Anspruch 1, wobei die Sensorbaugruppe aufweist:
ein Szintillatorarray (132), das Röntgenstrahlen in Licht umwandelt; und
ein Lichtdetektorarray (134), das Licht von dem Szintillatorarray empfängt und in ein analoges elektrisches Signal umwandelt.

6. Gerät nach Anspruch 4, wobei der umlaufende elektrische Schaltkreis die analogen elektrischen Signale von dem Lichtsensorarray an die Sensorsignalverarbeitungselektronik überträgt, und wobei die Sensorsignalverarbeitungselektronik die analogen elektrischen Signale in digitale Signale umwandelt.

7. Gerät nach Anspruch 1, wobei das strahlungsabschirmende Substrat aus mindestens einem von einem Metall und einer Metalllegierung besteht.

8. Gerät nach Anspruch 1, wobei das strahlungsabschirmende Substrat aus Wolfram besteht.

9. Gerät nach Anspruch 1, wobei die Sensorbaugruppe weniger als etwa 3 mm von der Sensorsignalverarbeitungselektronik entfernt positioniert ist.

10. Gerät nach Anspruch 1, wobei die flexible Leiterplatte mehrere Schaltungsschichten aufweist.

11. Gerät nach Anspruch 10, wobei eine der mehreren Schaltungsschichten eine Erdungsschicht aufweist.

12. Gerät nach Anspruch 11, wobei die Erdungsschicht mit dem strahlungsabschirmenden Substrat verbunden ist.

13. Aktive Abschirmung für ein Röntgen-Computertomographiegerät ("CT-Gerät"), aufweisend:
ein strahlungsabschirmendes Substrat (100); und
einen umlaufenden elektrischen Schaltkreis (110), der um das Substrat gewickelt ist,
wobei das strahlungsabschirmende Substrat eine erste Seite und eine zweite Seite aufweist, und der Abstand zwischen der ersten und der zweiten Fläche des strahlungsabschirmenden Substrats zwischen etwa 0,5 mm und 2 mm beträgt, und wobei der umlaufende elektrische Schaltkreis dazu ausgelegt ist, elektrische Signale entlang Leiterbahnen mit einer Länge von nicht mehr als 100 mm von einer Sensorbaugruppe (200), die neben der ersten Seite des Substrats positioniert ist, zu Signalverarbeitungselektronik, die neben der zweiten Seite des Substrats positioniert ist, zu leiten.

14. Aktive Abschirmung nach Anspruch 13, wobei das Substrat dazu ausgelegt ist, die Sensorbaugruppe und die Signalverarbeitungselektronik mechanisch zu tragen.

15. Aktive Abschirmung nach Anspruch 14, wobei das strahlungsabschirmende Substrat dazu ausgelegt ist, die neben der zweiten Seite des Substrats positionierte Signalverarbeitungselektronik vor einer auf der ersten Seite des Substrats positionierten Röntgenquelle abzuschirmen.

16. Verfahren zur Erzeugung digitaler Signale, die ein Bild eines Objekts darstellen, aufweisend:
Übertragen von Röntgenstrahlen (236A, 236B, 236C) durch das Objekt von einer Röntgenquelle (234) auf einer Seite des Objekts zu einer Sensorbaugruppe XX(130), die auf einer Seite einer aktiven Abschirmung (98) mit gegenüberliegenden ersten und zweiten Seiten getragen wird, wobei die aktive Abschirmung auf einer Seite des Objekts angeordnet ist, die der Seite, auf der sich die Röntgenquelle befindet, gegenüberliegt; und
Leiten der von der Sensorbaugruppe erzeugten analogen Signale entlang Leiterbahnen eines umlaufenden Schaltkreisabschnitts (110), wobei die Leiterbahnen eine Länge von nicht mehr als 100 mm haben, zu einer Signalverarbeitungselektronik (150), die auf der Seite der aktiven Abschirmung, die der Seite, die die Sensorbaugruppe trägt, gegenüberliegt, getragen wird und neben dieser positioniert ist, und wobei der Abstand zwischen der ersten und der zweiten Fläche der aktiven Abschirmung zwischen etwa 0,5 mm und 2 mm beträgt.

17. Verfahren nach Anspruch 16, wobei der umlaufende Schaltkreisabschnitt der aktiven Abschirmung um einen Röntgenstrahlen blockierenden Substratabschnitt der aktiven Abschirmung gewickelt ist.

## Revendications

1. Appareil à rayons X, comprenant :
un substrat de protection vis-à-vis du rayonnement (100) comportant un premier côté (102) et un second côté opposé (104) ;
un circuit électrique enveloppant (110) enveloppé autour du substrat ;
un ensemble de capteurs rayons X (130) adapté pour générer des signaux de capteur indicatifs de rayons X arrivant en incidence dessus, l'ensemble de capteurs étant connecté au circuit électrique enveloppant et positionné de manière à être adjacent au premier côté (102) du substrat ; et
un ensemble de traitement de signal de capteur (150) connecté au circuit électrique enveloppant et positionné de manière à être adjacent au second côté (104) du substrat,
dans lequel le circuit électrique enveloppant comprend une pluralité de pistes de circuit (113),
dans lequel chacune de la pluralité de pistes de circuit présente une longueur non supérieure à environ 100 mm, et
dans lequel la distance entre les première et seconde surfaces du substrat est entre environ 0,5 mm et 2 mm.

2. Appareil selon la revendication 1, dans lequel le circuit électrique enveloppant comprend une carte de circuit imprimé souple.

3. Appareil selon la revendication 1, dans lequel le circuit électrique enveloppant comprend une couche diélectrique (122) formée sur le substrat de protection vis-à-vis du rayonnement et une couche de circuit (124) formée sur la couche diélectrique.

4. Appareil selon la revendication 1, dans lequel le circuit électrique enveloppant recouvre sensiblement le premier côté et le second côté du substrat.

5. Appareil selon la revendication 1, dans lequel l'ensemble de capteurs comprend :
un réseau de scintillateurs (132) qui convertit les rayons X en lumière ; et
un réseau de détecteurs de lumière (134) qui reçoit la lumière en provenance du réseau de scintillateurs et qui la convertit en signal électrique analogique.

6. Appareil selon la revendication 4, dans lequel le circuit électrique enveloppant transmet les signaux électriques analogiques en provenance du réseau de capteurs de lumière à l'électronique de traitement de signal de capteur et dans lequel l'électronique de traitement de signal de capteur convertit les signaux électriques analogiques en signaux numériques.

7. Appareil selon la revendication 1, dans lequel le substrat de protection vis-à-vis du rayonnement est réalisé à partir d'au moins un matériau parmi un métal et un alliage de métal/métaux.

8. Appareil selon la revendication 1, dans lequel le substrat de protection vis-à-vis du rayonnement est réalisé à partir de tungstène.

9. Appareil selon la revendication 1, dans lequel l'ensemble de capteurs est positionné à moins d'environ 3 mm de l'électronique de traitement de signal de capteur.

10. Appareil selon la revendication 1, dans lequel la carte de circuit souple comprend une pluralité de couches de circuit.

11. Appareil selon la revendication 10, dans lequel l'une de la pluralité de couches de circuit comprend une couche de masse.

12. Appareil selon la revendication 11, dans lequel la couche de masse est connectée au substrat de protection vis-à-vis du rayonnement.

13. Protection active pour une machine de tomodensitométrie à rayons X ("machine CT") comprenant :
un substrat de protection vis-à-vis du rayonnement (100) ; et
un circuit électrique enveloppant (110) enveloppé autour du substrat,
dans laquelle le substrat de protection vis-à-vis du rayonnement comporte un premier côté et un second côté, et la distance entre les première et seconde surfaces du substrat de protection vis-à-vis du rayonnement est entre environ 0,5 mm et 2 mm, et dans lequel le circuit électrique enveloppant est adapté pour router des signaux électriques suivant des pistes de circuit présentant une longueur non supérieure à 100 mm depuis un ensemble de capteurs (200) positionné de manière à être adjacent au premier côté du substrat jusqu'à une électronique de traitement de signal positionnée de manière à être adjacente au second côté du substrat.

14. Protection active selon la revendication 13, dans laquelle le substrat est adapté pour supporter mécaniquement l'ensemble de capteurs et l'électronique de traitement de signal.

15. Protection active selon la revendication 14, dans laquelle le substrat de protection vis-à-vis du rayonnement est adapté pour protéger l'électronique de traitement de signal positionnée de manière à être adjacente au second côté du substrat vis-à-vis d'une source de rayons X positionnée sur le premier côté du substrat.

16. Procédé de génération de signaux numériques représentatifs d'une image d'un sujet comprenant :
l'émission de rayons X (236A, 236B, 236C) au travers du sujet depuis une source de rayons X (234) sur un côté du sujet jusqu'à un ensemble de capteurs (130) supporté sur un côté d'une protection active (98) comportant des premier et second côtés opposés, la protection active étant localisée sur un côté du sujet opposé au côté au niveau duquel la source de rayons X est localisée ; et
le routage de signaux analogiques générés par l'ensemble de capteurs suivant des pistes de circuit d'une partie de circuit enveloppante (110), les pistes de circuit présentant une longueur non supérieure à 100 mm jusqu'à une électronique de traitement de signal (150) supportée sur et positionnée de manière à être adjacente au côté de la protection active opposé au côté supportant l'ensemble de capteurs, et dans lequel la distance entre les première et seconde surfaces de la protection active est entre environ 0,5 mm et 2 mm.

17. Procédé selon la revendication 16, dans lequel la partie de circuit enveloppante de la protection active est enveloppée autour d'une partie de substrat de blocage de rayons X de la protection active.
